## Europäisches Patentamt
## European Patent Office
## Office européen des brevets

(11) Numéro de publication: **0 091 334**
**B1**

# FASCICULE DE BREVET EUROPÉEN

(45) Date de publication du fascicule du brevet: **06.06.88**

(51) Int. Cl.⁴: **A 61 F 9/00,** A 61 B 17/36

(21) Numéro de dépôt: **83400560.5**

(22) Date de dépôt: **17.03.83**

(54) **Appareil de chirurgie opthalmologique à laser.**

(30) Priorité: **01.04.82 FR 8205654**

(43) Date de publication de la demande:
**12.10.83 Bulletin 83/41**

(45) Mention de la délivrance du brevet:
**06.06.88 Bulletin 88/23**

(84) Etats contractants désignés:
**AT BE CH DE FR GB IT LI LU NL SE**

(56) Documents cité:
**EP-A-0 007 256**
**EP-A-0 076 740**
**WO-A-80/01642**
**FR-A-2 344 269**
**FR-A-2 344 270**
**US-A-3 403 957**

(73) Titulaire: **SYNTHELABO, 58, rue de la Glacière, F-75621 Paris Cedex 13 (FR)**

(72) Inventeur: **Tagnon, Luc, 11 Rue Brière de Boismont, F-94160 Saint Mande (FR)**

(74) Mandataire: **CABINET BONNET- THIRION, 95 Boulevard Beaumarchais, F-75003 Paris (FR)**

## Description

La présente invention concerne d'une manière générale les appareils de chirurgie ophtalmologique à laser du type de celui décrit dans la demande de brevet européen déposée le 7 Juin 1979 sous le No 79 400 370.7 et publiée comme EP-A-0 007 256 aussi bien que dans la demande de brevet européen déposée le 28 Septembre 1982 sous le No 82 401 760 et publiée comme EP-A-0 076 740.

Globalement, un tel appareil de chirurgie ophtalmologique comporte une unité laser, propre à l'émission d'au moins un faisceau laser, et une unité d'exploitation, propre à focaliser sur un point de visée ledit faisceau laser.

En pratique, l'unité laser comporte de préférence deux lasers, à savoir un laser principal opératoire de puissance suffisamment élevée pour que son faisceau intervienne non pas par transfert thermique, mais par claquage optique, et à cet égard un laser YAG en mode bloqué émettant dans l'infrarouge donne satisfaction, et un laser auxiliaire de visée à faisceau visible propre à matérialiser pour l'opérateur le point de visée, par exemple sous la forme d'une tache rouge de très faible diamètre.

Conjointement, l'unité d'exploitation comporte usuellement une lampe à fente.

Ainsi qu'on le sait, une telle lampe à fente comporte, globalement, sur un même châssis de support, d'une part un microscope, propre à permettre à l'opérateur d'observer le point de visée à traiter, et d'autre part un projecteur de fente, c'est-à-dire un dispositif optique lumineux propre à assurer la formation d'une fente lumineuse dans le plan de mise au point dudit microscope, pour faciliter l'observation du point de visée.

Pour adaptation notamment à la morphologie du patient à traiter, le châssis de support d'une telle lampe à fente est, dans son ensemble, et sous le contrôle de moyens de commande à la disposition de l'opérateur, monté réglable en position sur un socle, en pratique un socle plan, tant parallèlement à ce socle, dans deux directions orthogonales du plan de celui-ci, l'une correspondant à un réglage en profondeur, suivant l'axe de visée, l'autre, à un réglage latéral, perpendiculairement à cet axe de visée, que, pour un réglage en hauteur, perpendiculairement audit socle.

De manière classique, ces moyens de commande comportent d'une part un palonnier, ou levier basculant, propre au réglage en profondeur, lorsqu'il est basculé d'avant en arrière, et au réglage latéral, lorsqu'il est basculé de droite à gauche, et, d'autre part, une molette, qui, disposée à la base de ce palonnier, autour de celui-ci, permet le réglage en hauteur.

Il est clair que la manipulation simultanée d'un tel palonnier et d'une telle molette est difficile.

Dans la demande de brevet français publiée sous le N° 2 344 269, il est prévu, en sus, de monter rotatif autour d'un axe vertical et autour d'un axe horizontal, à l'aide d'un mécanisme de réglage de précision, l'organe de sortie du faisceau laser, ce qui rend encore plus ardue une éventuelle manipulation simultanée du palonnier, de la molette et de ce mécanisme de réglage de précision.

Corollairement, pour la focalisation sur le point de visée du ou des faisceaux laser émis par l'unité laser, l'unité d'exploitation comporte une lentille de focalisation, qui, par exemple, tel que décrit dans la demande de brevet européen N° 82 401 760 (EP-A-0 076 740) mentionnée ci-dessus, peut former la lentille de sortie de ladite unité laser.

En pratique, l'unité d'exploitation comporte également, en aval de sa lentille de focalisation, un miroir de renvoi apte à diriger sur le point de visée le ou les faisceaux laser émis par l'unité laser, ceux-ci arrivant usuellement selon l'axe de visée alors que l'unité laser est disposée latéralement par rapport à celui-ci.

De manière classique, cette lentille de focalisation et ce miroir de renvoi sont assujettis de manière fixe au châssis de support de la lampe à fente.

Or, une opération de chirurgie ophtalmologique implique normalement une intervention sur une pluralité de points distincts, échelonnés suivant une trajectoire déterminée.

Pour faire parcourir cette trajectoire au point de visée du ou des faisceaux laser, l'opérateur doit normalement à ce jour ajuster en permanence en conséquence la position du châssis de support de la lampe à fente par rapport à son socle.

Or, comme souligné ci-dessus, cet ajustement en position du châssis de support de la lampe à fente suppose à ce jour la manipulation simultanée d'un palonnier et d'une molette, et, nécessitant donc une grande coordination de mouvements, il est particulièrement malaisé à assurer de manière sûre.

La présente invention a d'une manière générale pour objet une disposition permettant au contraire de faciliter le respect d'une trajectoire pour le point de visée.

De manière plus précise, elle a pour objet un appareil de chirurgie ophtalmologique du genre comportant une unité laser, propre à l'émission d'au moins un faisceau laser, et une unité d'exploitation, propre à focaliser sur un point de visée ledit faisceau laser, ladite unité d'exploitation comportant à cet effet un chassis, dit ci-après par simple commodité châssis de support, monté réglable en position sur un socle sous le contrôle, notamment, d'un palonnier, qui, à la disposition de l'opérateur, est propre à un déplacement dudit chassis de support parallèlement audit socle, et, sur ledit châssis de support, d'une part, pour la focalisation recherchée du faisceau laser, une lentille de focalisation, et, en aval de celle-ci, un miroir de renvoi, et, d'autre part, pour l'observation du point de visée, une lampe à fente, cet appareil de chirurgie ophtalmologique étant d'une manière générale caractérisé en ce que ladite lentille de focalisation et ledit miroir de renvoi sont solidarisés entre eux en service, en étant conjointement portés par (ou liés en mouvement à) un équipage, qui, lui-même porté par le châssis de support, est monté mobile vis-à-vis de celui-ci, perpendiculairement au socle, et sous le contrôle de moyens de commande distincts de ceux associés audit châssis de support, lesdits moyens de commande comportant un moteur, qui est porté par le châssis de support, et dont l'alimentation électrique est asservie à un organe de mise en oeuvre accessible du palonnier.

Par exemple, cet organe de mise en oeuvre est porté par le palonnier que comportent les moyens de commande propres au châssis de support.

En variante, il est indépendant de ce palonnier, mais disposé au voisinage de celui-ci.

Quoi qu'il en soit, pour le respect d'une trajectoire pour le point de visée il est possible, dans l'un et l'autre cas, d'agir d'une seule main, d'une part sur le palonnier, pour le réglage en profondeur et le réglage latéral éventuellement nécessaires, et, d'autre part, sur l'organe de mise en oeuvre des moyens de commande propres à l'équipage mobile portant la lentille de focalisation et le miroir de renvoi, pour le réglage en hauteur également éventuellement nécessaire.

Une telle action simultanée sur un palonnier et un organe de mise en oeuvre est particulièrement aisée.

Certes, lorsque, comme décrit dans la demande de brevet européen No 82 401 760 mentionnée ci-dessus, la lentille de focalisation de l'unité d'exploitation forme conjointement la lentille de sortie de l'unité laser, son déplacement conduit à une défocalisation du ou des faisceaux émis par cette unité laser.

Mais, compte tenu de la longueur du chemin optique en amont de la lentille de focalisation, d'une part, et du déplacement relativement très limité de celle-ci pour le réglage en hauteur à assurer, d'autre part, cette défocalisation est quasi insensible et n'a avantageusement aucun effet pratique.

Les caractéristiques et avantages de l'invention ressortiront d'ailleurs de la description qui va suivre, à titre d'exemple, en référence aux dessins schématiques annexés sur lesquels:

la figure 1 est, en perspective, un bloc diagramme d'un appareil de chirurgie ophtalmologique suivant l'invention;

la figure 2 en est une vue partielle en élévation-coupe de l'unité d'exploitation;

la figure 3 reprend à échelle supérieure, une partie de la figure 2 repérée par un encart III sur celle-ci;

la figure 4 est une vue transversale en coupe, suivant la ligne IV-IV de la figure 3;

les figures 5 et 6 sont, avec des arrachements locaux, des vues latérales partielles, suivant respectivement la flèche V et la flèche VI de la figure 3;

la figure 7 est une vue partielle en élévation, suivant la flèche VII de la figure 2;

la figure 8 est une vue analogue à celle de la figure 7 et concerne une variante de réalisation.

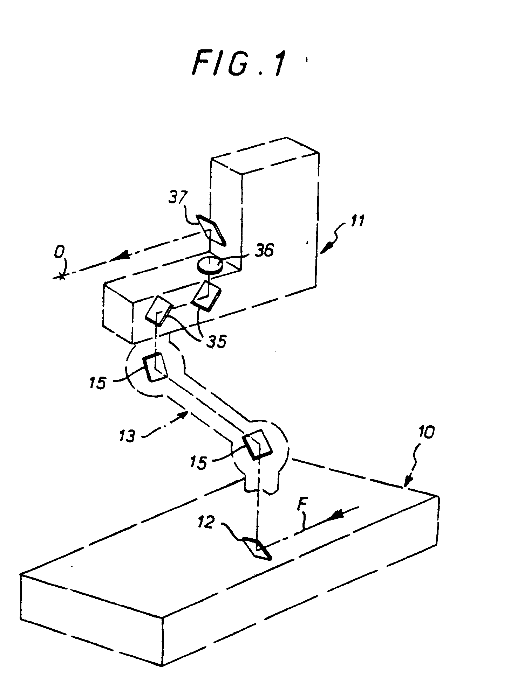

Tel que schématisé sur la figure 1, l'appareil de chirurgie ophtalmologique suivant l'invention comporte globalement, d'une manière connue en soi, une unité laser 10, propre à l'émission d'au moins un faisceau laser F, schématisé en traits interrompus sur la figure 1, et une unité d'exploitation 11, propre, notamment, à focaliser sur un point de visée 0 ledit faisceau laser F.

L'unité laser 10 ne faisant pas partie de la présente invention, elle ne sera pas décrite ici.

Elle peut par exemple être du type de celle décrite dans la demande de brevet européen No 82 401 760 mentionnée ci-dessus.

Sur la figure 1, seul en a été représenté, schématiquement, le miroir de renvoi de sortie 12.

En pratique, l'unité d'exploitation 11 devant pouvoir être ajustée en position suivant trois directions orthogonales, ainsi qu'il apparaîtra ci-après, et l'unité laser 10 étant au contraire fixe, la liaison entre cette unité laser 10 et cette unité d'exploitation 11 se fait par l'intermédiaire d'un bras de transfert 13 propre à permettre une telle capacité de mouvement relatif entre lesdites unités.

Un tel bras de transfert 13 ne fait pas partie non plus en lui-même de la présente invention, et il ne sera donc pas décrit plus en détail ici.

Il peut par exemple être du type de celui décrit dans la demande de brevet français déposée le 22 Décembre 1981 sous le No 81 23947 et publiée sous le No 2 518 764.

Il comporte une pluralité de miroirs de renvoi.

Seuls, sur la figure 1, en ont été schématisés les miroirs de renvoi d'extrémité 15.

L'unité d'exploitation 11 ne sera pas non plus décrite dans tous ses détails ici.

Seuls en seront décrits les constituants nécessaires à la compréhension de l'invention.

En pratique, cette unité d'exploitation 11 comporte, sur un châssis de support dont tous les éléments seront indifféremment repérés par la même référence 16, une lampe à fente 17, constituée, de manière connue en soi, d'une part d'un microscope 18, propre à l'observation du point de visée 0 à traiter, et d'autre part d'un projecteur de fente 20, propre à la formation, dans le plan de mine au point du microscope 18, d'une fente lumineuse, en pratique verticale, destinée à faciliter l'observation dudit point de visée 0.

Ce point de visée 0 appartient à l'oeil du patient à traiter, dont la position est déterminée par un appui frontal 21 et une mentonnière 22 montés réglables verticalement en position sur des colonnettes 23 elles-mêmes portées rigidement par un socle 24.

Suivant la morphologie de ce patient, il est nécessaire d'ajuster en position la lampe à fente 17.

Le châssis de support 16 portant celle-ci est donc monté réglable en position sur le socle 24, sous le contrôle de moyens de commande à la disposition de l'opérateur.

En pratique, et de manière connue en soi, le châssis de support 16 est monté réglable en hauteur sur une semelle 25, et celle-ci porte sur le socle 24, d'une part, à l'avant, par deux galets 26, qui engrènent chacun respectivement avec des rails à picots 27 appartenant au socle 24, et qui sont conjointement montés coulissants sur une même barre transversale 28 appartenant au châssis de support 16, et, d'autre part, à l'arrière, par une bille 30.

Comme mentionné ci-dessus, ces dispositions sont bien connues en elles-mêmes, et, ne faisant pas partie de la présente invention, elles ne seront pas décrites en détail ici.

Il suffira de préciser que les moyens de commande associés au châssis de support 16 pour son réglage en position comportent, d'une part, pour un déplacement parallèlement au socle 24, un palonnier 32, qui, basculé d'avant en arrière, permet un réglage en profondeur, suivant l'axe de visée, et qui, basculé de droite à gauche, permet un réglage latéral, perpendiculairement à cet axe, et, d'autre part, à la base de ce palonnier 32, constitué en pratique d'un simple levier monté basculant, pour un déplacement perpendiculairement au socle 24, une molette 33, qui, par rotation dans un sens ou dans l'autre, permet un réglage en hauteur.

Au châssis de support 16 est ajustée, à la base de celui-ci, l'extrémité, qui est verticale, du bras de transfert 13.

Outre, dans la forme de réalisation représentée, deux miroirs de renvoi 35, l'unité d'exploitation 11 comporte, pour la focalisation recherchée du faisceau laser F, une lentille de focalisation 36, et, en aval de celle-ci, pour un repliement à 90° de ce faisceau laser F, un miroir de renvoi 37.

Tel que décrit dans la demande de brevet européen No 82 401 760 mentionnée ci-dessus, la lentille de focalisation 36 peut avantageusement constituer conjointement la lentille de sortie du ou des expandeurs de faisceau que comporte usuellement l'unité laser 10.

Quoi qu'il en soit, cette lentille de focalisation 36, et le miroir de renvoi 37 qui lui fait suite, sont, de manière usuelle, disposés en bout d'un tube 38 formant un prolongement, dans l'unité d'exploitation 11, du bras de transfert 13.

Comme l'extrémité de ce dernier, ce tube 38 est solidaire du châssis de support 16.

Suivant l'invention, la lentille de focalisation 36 et le miroir de renvoi 37 sont conjointement portés par un équipage 40, qui, lui-même porté par le châssis de support 16, et, plus précisément, par le tube 38 solidaire de celui-ci, est monté mobile vis-à-vis de ce châssis de support 16, sous le contrôle de moyens de commande détaillés ci-après distincts de ceux associés à ce dernier.

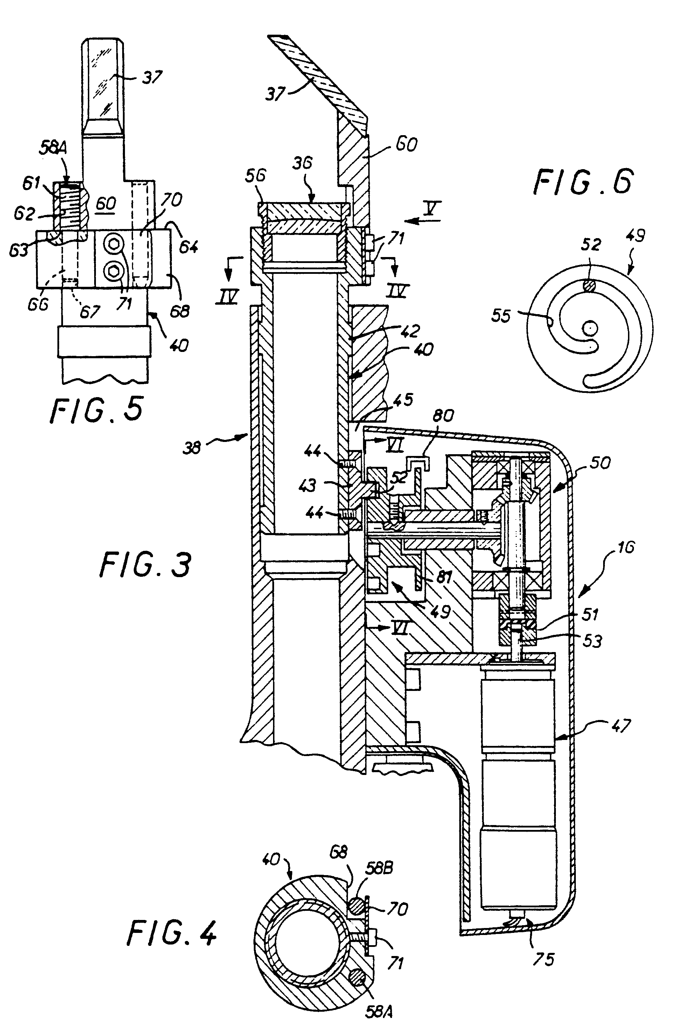

En pratique, l'équipage 40 portant ainsi la lentille de focalisation 36 et le miroir de renvoi 37 est constitué par un tube, monté coulissant axialement dans le tube 38, figure 3.

Autrement dit, cet équipage 40 forme un coulisseau monté mobile dans une glissière formée par le tube 38.

Il est donc monté mobile suivant l'axe optique de la lentille de focalisation 36, perpendiculairement au socle 24 portant le châssis de support 16.

En pratique, pour son coulissement dans le tube 38, le tube constituant l'équipage 40 présente, à distance l'un de l'autre, deux bossages annulaires 42, par lesquels il porte sur la surface intérieure dudit tube 38.

En outre, pour son blocage en rotation sur le tube 38, il porte, longitudinalement, à sa base, une barrette 43, qui lui est par exemple rapportée par des vis 44, et qui est engagée à coulissement dans une fente 45 ménagée à cet effet dans le tube 38.

Suivant l'invention, les moyens de commande associés à l'équipage 40 comportent un moteur 47, qui est porté par le châssis de support 16, et dont l'alimentation électrique est asservie, tel que détaillé ci-après, à un organe de mise en oeuvre 48 à la disposition de l'opérateur.

En pratique, dans la forme de réalisation représentée, l'équipage 40 porte en saillie, sur la barrette 43 dont il est muni, un ergot 52, par lequel il est en prise avec une came 49, qui, montée rotative sur le châssis de support 16, est, par l'intermédiaire d'un renvoi d'angle 50 et d'un joint élastique 51, calée en rotation sur l'arbre de sortie 53 du moteur 47.

Les modalités constructives correspondantes relèvent de l'homme de l'art et elles ne seront donc pas décrites en détail ici.

Tel qu'il est mieux visible à la figure 6, la came 49 est, dans la forme de réalisation représentée, une came dont la piste est formée par une rainure en spirale 55, avec laquelle est en prise l'ergot 52 porté par l'équipage 40.

Les extrémités de cette rainure en spirale 55 sont borgnes, et elles sont décalées radialement l'une par rapport à l'autre, sur un même rayon dans la forme de réalisation représentée.

Ainsi qu'il est aisé de le comprendre, compte tenu du blocage en rotation de l'équipage 40 sur le tube 38 porté par le châssis de support 16, une rotation, dans un sens, ou dans l'autre, de la came 49, entraîne un mouvement axial, de montée, ou de descente, de cet équipage 40 dans ce tube 38.

De manière connue en soi, la lentille de focalisation 36, qui peut être constituée par une lentille unique, un doublet, ou un quelconque groupement de lentilles, est portée par une bague 56 rapportée par vissage à l'extrémité du tube constituant l'équipage 40.

Quant au miroir de renvoi 37, il est, dans la forme de réalisation représentée, porté par l'équipage 40 par l'intermédiaire de deux tiges de centrage 58A, 58B parallèles à l'axe de la lentille de focalisation 36, et donc, à l'axe du tube constituant cet équipage 40.

Ces tiges de centrage 58A, 58B sont l'une et l'autre solidaires d'un bloc 60 portant le miroir de renvoi 37.

La tige de centrage 58A présente une tête filetée 61, par laquelle elle est engagée à vissage dans un perçage taraudé 62 de ce bloc 60, et, au-delà d'un épaulement 63, qu'elle présente transversalement, et par lequel elle porte sur la face transversale d'extrémité 64 de l'équipage 40, elle présente un fût 66, par lequel elle est simplement engagée à coulissement dans un perçage 67 de cet équipage 40.

La tige de centrage 58B, par contre, est simplement maintenue en appui contre un méplat 68 de l'équipage 40, faisant plan de référence, par des moyens élastiques, constitués, dans la forme de réalisation représentée, d'une simple lame de ressort 70 fixée par des vis 71 à cet équipage 40.

Ainsi qu'il est aisé de le comprendre, en faisant tourner la tige de centrage 58A à l'aide d'un tournevis, il est possible de déplacer le miroir de renvoi 37 parallèlement à l'axe du tube constituant l'équipage 40, pour un réglage en hauteur de ce miroir de renvoi 37 vis-à-vis de cet équipage 40.

Devant tourner dans un sens ou dans l'autre, le moteur 47, qui est en fait un moto-réducteur, est en pratique un moteur à courant continu.

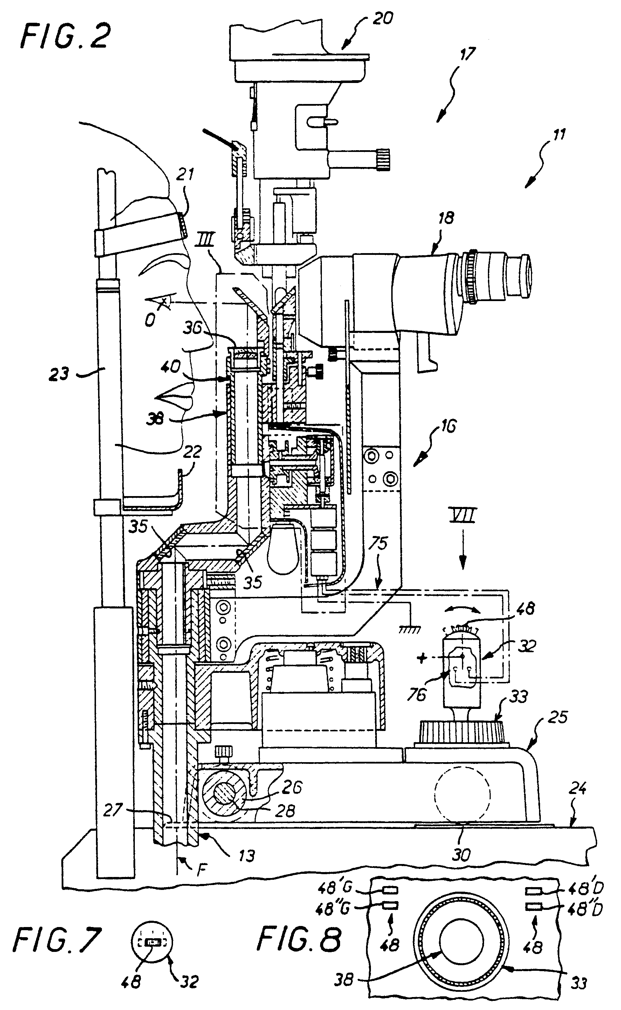

A la figure 2, on a schématiquement représenté en traits interrompus la filerie 75 nécessaire à sa desserte.

En bref, de manière connue en soi, alors qu'une de ses bornes est par exemple en permanence reliée à la masse, une tension positive est susceptible d'être alternativement reliée à l'une ou l'autre de ses autres bornes.

Dans la forme de réalisation représentée sur les figures 1 à 7, l'alimentation ainsi alternée du moteur 47 est sous le contrôle d'un inverseur 76, lui-même commandé par l'organe de mise en oeuvre 48 prévu suivant l'invention.

En outre, dans cette forme de réalisation, l'organe de mise en oeuvre 48 est porté par le palonnier 32, en bout de celui-ci, et il est constitué par un bouton à trois positions.

L'inverseur 76 est de même logé dans le palonnier 32.

Les modalités de construction nécessaires, ainsi que celles relatives à la liaison à établir entre l'organe de mise en oeuvre 48 et l'inverseur 76, relèvent de l'homme de l'art, et elles ne seront donc pas décrites en détail ici.

Quoi qu'il en soit, pour la position centrale, représentée en trait plein sur les figures 2 et 7, du bouton constituant l'organe de mise en oeuvre 48, le contact mobile de l'inverseur 76 est lui-même en position centrale neutre.

Le moteur 47, non alimenté, est dès lors inactif.

Par contre, pour l'une ou l'autre des positions d'extrémité, schématisées en traits interrompus sur les figures 2 et 7, du bouton constituant l'organe de mise en oeuvre 48, le contact mobile de l'inverseur 76, qui est relié à la source de tension positive correspondante, se trouve lui-même dans l'une ou l'autre de ses positions d'extrémité, et le moteur 47 se trouve alimenté, dans un sens pour l'une desdites positions d'extrémité, et dans l'autre pour l'autre de celles-ci.

L'équipage 40 portant suivant l'invention la lentille de focalisation 36 et le miroir de renvoi 37 se déplace dès lors parallèlement à son axe, vers le haut ou vers le bas.

Pour faire suivre une trajectoire au point de visée 0, il suffit donc, à l'opérateur, tout en agissant sur le palonnier 32 pour le réglage en profondeur et le réglage latéral éventuellement nécessaires, d'agir conjointement du pouce sur le bouton constituant l'organe de mise en oeuvre 48 dans un sens correspondant au réglage en hauteur également éventuellement nécessaire.

Suivant des modalités connues en elles-mêmes, ce bouton peut être si désiré un bouton à relâchement, c'est-à-dire un bouton à retour automatique à sa position centrale neutre, dès qu'une action préalablement exercée sur lui est relâchée.

Suivant la variante de réalisation schématiquement représentée à la figure 8, l'organe de mise en oeuvre 48 suivant l'invention comporte au moins un doublet de deux boutons poussoirs 48', 48'', qui, indépendants du palonnier 32, mais disposés au voisinage de celui-ci, par exemple, et tel que représenté, sur la semelle 25, à proximité immédiate de la molette 38, sont accessibles d'un doigt à l'opérateur, alors même que celui-ci, comme précédemment, tient et manipule de la même main, par exemple entre le pouce et l'index, ledit palonnier 32.

En pratique, dans la forme de réalisation représentée, deux doublets de tels boutons poussoirs sont prévus, l'un 48'D, 48''D, pour un droitier, à droite de la molette 33, l'autre 48'G, 48''G, pour un gaucher, à gauche de celle-ci.

De préférence, des boutons poussoirs sont des boutons poussoirs à relâchement ou sensitifs, dont l'intervention ne se poursuit que pour autant qu'une action est exercée sur eux, et, tel que représenté, ils sont légèrement décalés vers l'avant par rapport à la molette 33, et donc par rapport au palonnier 32, pour en faciliter l'accessibilité par rapport à celui-ci.

Lorsqu'une action, d'enfoncement par exemple, est exercée sur un bouton poussoir 48'D, ou 48'G, c'est l'une des bornes du moteur 47 qui se trouve reliée à la source de tension positive correspondante, et c'est l'autre de celles-ci lorsque, en alternative, une telle action est exercée sur un bouton poussoir 48''D, ou 48''G.

Comme précédemment, le moteur 47 se trouve ainsi commandé dans un sens ou dans l'autre, et donc l'équipage 40 déplacé vers le haut ou vers le bas.

Suivant une autre variante de réalisation, non représentée, l'organe de mise en oeuvre 48 est un bouton à affichage, une chaîne d'asservissement prévue à cet effet ajustant automatiquement la position de l'équipage 40 en fonction de celle d'un tel bouton à affichage.

En outre, suivant un développement de l'invention, l'alimentation électrique du moteur 47 est de préférence également asservie à des moyens de remise à zéro automatique en fin d'opération.

Par exemple, et tel que schématisé à la figure 3, sur laquelle seule l'une d'elles a été représentée, trois cellules opto-électriques 80 peuvent être prévues à cet effet autour d'un disque 81 qui, pour l'excitation de celles-ci, porte un repère.

L'une de ces cellules opto-électriques 80 occupe, autour du disque 81, une position image de la position milieu de l'équipage 40, tandis que les deux autres occupent chacune respectivement des positions images des positions d'extrémité de celui-ci.

Sous le contrôle d'un bouton spécial de déclenchement, qui peut par exemple être asservi à l'organe assurant usuellement la condamnation de la pédale de tir de l'unité laser 10 en fin d'opération, une chaîne logique, pilotée par les cellules opto-électrique 80, déclenche l'alimentation, systématiquement dans un sens, du moteur 47.

Si, au cours de la rotation correspondante du disque 81, le repère porté par celui-ci défile au droit de la cellule opto-électrique 80 dont la position est à l'image de la position milieu de l'équipage 40, cette chaîne logique arrête alors l'alimentation du moteur 47.

Si, par contre, ce repère vient à défiler au droit de l'une ou l'autre des deux autres cellules opto-électriques 80, la chaîne logique déclenche une inversion de l'alimentation du moteur 47, pour inversion du mouvement de déplacement de l'équipage 40.

L'arrivée de celui-ci à son point milieu déclenche ensuite, comme précédemment, par la cellule opto-électrique 80 concernée, l'arrêt de l'alimentation électrique du moteur 47.

Les modalites constructives correspondantes, qui peuvent être variées, relèvent de l'homme de l'art, et elles ne seront donc pas décrites en détail ici.

En variante, ou en complément, un bouton peut être prévu pour une remise à zéro manuelle du moteur électrique 47, si désiré.

La présente invention ne se limite d'ailleurs pas aux formes de réalisation et de mise en oeuvre décrites et représentées, mais englobe toute variante d'exécution conforme à l'objet des revendications ci-jointes.

## Revendications

1. Appareil de chirurgie ophtalmologique du genre comportant une unité laser (10), propre à l'émission d'au moins un faisceau laser (F), et une unité d'exploitation (11) propre à focaliser sur un point de visée (0) ledit faisceau laser (F), ladite unité d'exploitation (11) comportant à cet effet un châssis (16), dit ci-après par simple commodité chassis de support, monté réglable en position sur un socle (24) sous le contrôle, notamment, d'un palonnier (32), qui, à la disposition de l'opérateur, est propre à un déplacement dudit chassis de support (16) parallèlement audit socle (24), et, sur ledit châssis de support (16), d'une part, pour la focalisation recherchée du faisceau laser (F), une lentille de focalisation (36), et, en aval de celle-ci, un miroir de renvoi (37), et, d'autre part, pour l'observation du point de visée (0), une lampe à fente (17), caractérisé en ce que ladite lentille de focalisation (36) et ledit miroir de renvoi (37) sont solidarisés entre eux en service, en étant conjointement portés par, ou liés en mouvement à, un équipage (40), qui, lui-même porté par le châssis de support (16), est monté mobile vis-à-vis de celui-ci, perpendiculairement au socle (24), et sous le contrôle de moyens de commande distincts de ceux (32, 33) associés au dit châssis de support (16), lesdits moyens de commande comportant un moteur (47), qui est porté par le châssis de support (16), et dont l'alimentation électrique est asservie à un organe de mise en oeuvre (48) accessible du palonnier (32).

2. Appareil de chirurgie ophtalmologique suivant la revendication 1, caractérisé en ce que l'équipage (40) portant la lentille de focalisation (36) et le miroir de renvoi (37) est monté mobile suivant l'axe optique de ladite lentille de focalisation (36).

3. Appareil de chirurgie ophtalmologique suivant l'une quelconque des revendications 1, 2, caractérisé en ce que l'équipage (40) portant la lentille de focalisation (36) et le miroir de renvoi (37) forme un coulisseau monté mobile dans une glissière et il porte en saillie un ergot (52) par lequel il est en prise avec une came (49) calée en rotation sur l'arbre du moteur (47).

4. Appareil de chirurgie ophtalmologique suivant l'une quelconque des revendications 1 à 3, caractérisé en ce que l'organe de mise en oeuvre (48) auquel est asservie l'alimentation électrique du moteur (47) est porté par le palonnier (32).

5. Appareil de chirurgie ophtalmologique suivant la revendication 4, caractérisé en ce que ledit organe de mise en oeuvre (48) est un bouton à trois positions disposé en bout du palonnier (32).

6. Appareil de chirurgie ophtalmologique suivant l'une quelconque des revendications 1 à 3, caractérisé en ce que l'organe de mise en oeuvre (48) auquel est as servie l'alimentation électrique du moteur (47) est indépendant dudit palonnier (32).

7. Appareil de chirurgie ophtalmologique suivant la revendication 1, caractérisé en ce que l'alimentation électrique du moteur (47) est en outre asservie à des moyens de remise à zéro automatique en fin d'opération.

8. Appareil de chirurgie ophtalmologique suivant la revendication 1, caractérisé en ce que le miroir de renvoi (37) est porté par l'équipage mobile (40) par l'intermédiaire de deux tiges de centrage (58A, 58B) parallèles à l'axe de la lentille de focalisation (36) dont l'une (58A) est engagée à coulissement dans un perçage (67) dudit équipage mobile (40) et dont l'autre (58B) est simplement maintenue en appui contre un méplat (68) de celui-ci par des moyens élastiques (70).

9. Appareil de chirurgie ophtalmologique suivant la revendication 8, caractérisé en ce que, le miroir de renvoi (37) étant porté par un bloc (68), la tige de centrage (58A) qui lui est associée présente une tête filetée (61), qui est engagée à vissage dans un perçage taraudé (62) dudit bloc (60), et qui présente transversalement un épaulement (63) par lequel elle porte sur l'équipage mobile (40).

**Patentansprüche**

1. Vorrichtung zur Augenoperation mit einer Lasereinrichtung (10), die zumindest einen Laserstrahl (F) aussenden kann, und einer Auswerteeinrichtung (11), die den Laserstrahl (F) auf einen Zielpunkt (0) fokussieren kann und zu diesem Zweck umfaßt ein Gestell (16), nachfolgend einfach Traggestell genannt, welches in seiner Position auf einem Grundgestell (24) unter der Steuerung, insbesondere eines Steuerhebels (32) einstellbar angebracht ist, der zur Verfügung der Bedienungsperson steht und für eine Verschiebung des Traggestells (16) parallel zum Grundgestell (24) geeignet ist, und auf dem Traggestell (16) einerseits für die gesuchte Fokussierung des Laserstrahls (F) eine Fokussierungslinse (36) und, in Strahlungsrichtung hinter dieser, einen Reflektionsspiegel (37) und andererseits für die Beobachtung des Zielpunktes (0) eine Spaltlampe (17), dadurch gekennzeichnet, daß die Fokussierungslinse (36) und der Reflektionsspiegel (37) im Betrieb miteinander fest verbunden sind, indem sie zusammen von einem beweglichen Organ (40) getragen werden oder mit diesem bewegungsmäßig verbunden sind, welches seinerseits von dem Traggestell (16) getragen gegenüber diesem senkrecht zum Grundgestell (24) und unter der Steuerung von diesen getrennten Steuermitteln (32, 33), die dem Traggestell (16) zugeordnet sind, beweglich angebracht ist, wobei die Steuermittel einen Motor (47) umfassen, der von dem Traggestell (16) getragen wird und dessen elektrische versorgung von einem Einschaltorgan (48) gesteuert wird, welches vom Steuerhebel (32) zugänglich ist.

2. Vorrichtung zur Augenoperation nach Anspruch 1, dadurch gekennzeichnet, daß das bewegliche Organ (40), welches die Fokussierungslinse (36) und den Reflektionsspiegel (37) trägt, entlang der optischen Achse der Fokussierungslinse (36) beweglich angebracht ist.

3. Vorrichtung zur Augenoperation nach Anspruch 1 oder 2, dadurch gekennzeichnet, daß das bewegliche Organ (40), welches die Fokussierungslinse (36) und den Reflektionsspiegel (37) trägt, einen Schlitten bildet, der in einer Gleitschiene beweglich angebracht ist, und daß es einen Zapfen (52) vorspringend trägt, mit welchem es mit einem Hocken (49) in Eingriff steht, der auf der Welle des Motors (47) drehfest verkeilt ist.

4. Vorrichtung zur Augenoperation nach einem der Ansprüche 1 bis 3, dadurch gekennzeichnet, daß das Einschaltorgan (48), über das die elektrische Versorgung des Motors (47) gesteuert wird, von dem Steuerhebel (32) getragen wird.

5. Vorrichtung zur Augenoperation nach Anspruch 4, dadurch gekennnzeichnet, daß das Einschaltorgan (48) ein Dreistellungsschalter ist, der auf dem Kopfende des Steuerhebels (32) angeordnet ist.

6. Vorrichtung zur Augenoperation nach einem der Ansprüche 1 bis 3, dadurch gekennzeichnet, daß das Einschaltorgan (48), mit welchem die elektrische Versorgung (47) gesteuert wird, von dem Steuerhebel (32) unabhängig ist.

7. Vorrichtung zur Augenoperation nach Anspruch 1, dadurch gekennzeichnet, daß die elektrische Versorgung des Motors (47) außerdem von Mitteln für die automatische Nullstellung am Betriebsende gesteuert wird.

8. Vorrichtung zur Augenoperation nach Anspruch 1, dadurch gekennzeichnet, daß der Reflektionsspiegel (37) von dem beweglichen Organ (40) mittels zweier zur Achse der Fokussierungslinse (36) paralleler zentrierstangen (58a, 58b) getragen wird, von denen eine (58a) in einer Bohrung (67) des beweglichen Organs (40) gleitend in Eingriff steht und die andere (58b) einfach gegen eine flache Stelle (68) von diesem durch elastische Mittel (70) abgestützt gehalten wird.

9. Vorrichtung zur Augenoperation nach Anspruch 8, dadurch gekennzeichnet, daß bei von einem Block (60) getragenem Reflektionsspiegel (37) die diesem zugeordnete Zentrierstange (58a) einen Gewindekopf (61), welcher in einer mit Gewinde versehenen Bohrung (62) des Blockes (60) in Gewindeeingriff steht, aufweist und in Querrichtung eine Schulter (63) umfaßt, mit der sie sich auf dem beweglichen Organ (40) abstützt.

**Claims**

1. An ophthalmological surgical apparatus of the type comprising a laser unit (10) for emitting at least one laser beam (F) and an operating unit (11) for focussing said laser beam (F) on to a target point (0), said operating unit (11) comprising for that purpose a frame structure (16), referred to hereinafter for the sake of convenience as the support frame structurre, mounted adjustably in position on a base (24) under the control in particular of a swing lever (32) which, controlled by the operator, is operable to cause displacement of said support frame structure (6) parallel to said base (24) and, on said support frame structure (16), on the one hand, for the desired focussing of the laser beam (F), a focussing lens (36) and, downstream thereof, a reflecting mirror (37), and, on the other hand, for observation of the target point (O), a slit-type lamp (17), characterised in that said focussing lens (36) and said reflecting mirror (37) are fixed relative to each other in operation by being jointly carried by or connected in respect of movement thereof to an assembly (40) which, itself carried by the support frame structure (16), is mounted movably with respect thereto perpendicularly to the base (24) and under the control of control means separate from those (32, 33) associated with said support frame structure (16), said control means comprising a motor (47) which is carried by the support frame structure (16) and the supply of electrical power to which is controlled by an operating member (48) accessible from the swing lever (32).

2. Ophthalmological surgical apparatus according to claim 1 characterised in that the assembly (40) carrying the focussing lens (36) and the reflecting mirror (37) is mounted movably along the optical axis of said fusing lens (36).

3. Ophthalmological surgical apparatus according to either one of claims 1 and 2 characterised in that the assembly (40) carrying the focussing lens (36) and the reflecting mirror (37) forms a slider which is mounted movably in a slide and it carries in projecting relationship a lug (52) by way of which it is engaged with a cam (49) which is non-rotatably fixed on the shaft of the motor (47).

4. Ophthalmological surgical apparatus according to any one of claims 1 to 3 characterised in that the operating member (48) for controlling the supply of electrical power to the motor (47) is carried by the swing lever (32).

5. Ophthalmological surgical apparatus according to claim 4 characterised in that said operating member (48) is a button having three positions, disposed at the end of the swing lever (32).

6. Ophthalmological surgical apparatus according to any one of claims 1 to 3 characterised in that the operating member (48) for controlling the supply of electrical power to the motor (47) is independent of said swing lever (32).

7. Ophthalmological surgical apparatus according to claim 1 characterised in that the supply of electrical power to the motor (47) is also controlled by means for automatic resetting to zero at the end of an operation.

8. Ophthalmological surgical apparatus according to claim 1 characterised in that the reflecting mirror (37) is carried by the movable assembly (40) by means of two centering rods (58A, 58B) which are parallel to the axis of the focussing lens (36) and of which one (58A) is engaged slidably in an aperture (67) in said cable assembly (40) while the other (58B) is simply held in a position of bearing against a flat (68) thereon by resilient means (70).

9. Ophthalmological surgical apparatus according to claim 8 characterised in that, the reflecting mirror (37) being carried by a block (68), the centering rod (58A) which is associated therewith has a screwthreaded head (61) which is engaged by screwing in a threaded hole (62) in said block (60), and which transversely has a shoulder (63) by way of which it bears on the movable assembly (40).

# FIG.1

FIG.2

FIG.7

FIG.8

0 091 334

FIG.5

FIG.3

FIG.4

FIG.6

5